# EUROPÄISCHE PATENTANMELDUNG

(11) **EP 4 600 367 A1**
(43) Veröffentlichungstag der Anmeldung: **13.08.2025**
(21) Anmeldenummer: 24156272.7
(22) Anmeldetag: 07.02.2024
(51) Int. Cl.: C12P 13/00, C12N 9/06

(54) **ENZYMATISCHE SPALTUNG VON N-ALKYLVERBINDUNGEN NACH DER CHEMOLYSE VON POLYURETHANEN**

(71) Anmelder: Covestro Deutschland AG, 51373 Leverkusen (DE)
(72) Erfinder: Schaffert, Lena, 51375 Leverkusen (DE); Reisky, Lukas, 50996 Köln (DE); Hinzmann, Dirk, 50259 Pulheim (DE); Goebbels, Simone, 41352 Korschenbroich (DE); Wershofen, Stefan, 41065 Mönchengladbach (DE)
(74) Vertreter: Levpat

(57) **Zusammenfassung**

Die vorliegende Erfindung betrifft die enzymatische Spaltung von sekundären Aminen, welche bei der Chemolyse von Urethanen anfallen.

## Beschreibung

Die vorliegende Erfindung betrifft die enzymatische Spaltung von sekundären Aminen, welche bei der Chemolyse von Urethanen anfallen.

Polyurethanschäume finden vielfältige Anwendungen in der Industrie und im Alltag. Üblicherweise wird zwischen Polyurethanschäumen und sog. "CASE"-Produkten unterschieden, wobei "CASE" ein Sammelbegriff für *Polyurethan-Beschichtungen* (z. B. Lacke), *-Klebstoffe, -Dichtstoffe* und *-Elastomere* ist. Die Polyurethanschäume werden üblicherweise in Hartschäume und Weichschäume unterteilt. All diesen Produkten ist trotz ihrer Verschiedenheit die Polyurethangrundstruktur gemeinsam, die durch die Polyadditionsreaktion eines mehrwertigen Isocyanats und eines Polyols entsteht und sich beispielsweise für ein Polyurethan, das auf einem Diisocyanat **O=C=N-R-N=C=O** und einem Diol H-O-R`-O-H basiert (wobei R und R` organische Reste bezeichnen) als

**~~~[O-R'-O-(O=C)-HN-R-NH-(C=O)]~~~**

darstellen lässt.

Gerade wegen des großen wirtschaftlichen Erfolges der Polyurethanprodukte fallen auch große Mengen an Polyurethanabfall (z. B. aus alten Matratzen oder Sitzmöbeln) an, der einer sinnvollen Verwendung zugeführt werden muss. Die technisch am einfachsten umzusetzende Art der Wiederverwendung ist die Verbrennung unter Nutzung der freiwerdenden Verbrennungswärme für andere Prozesse, beispielweise industrielle Herstellprozesse. Auf diese Weise ist jedoch eine Schließung der Rohstoffkreisläufe nicht möglich. Eine andere Art der Wiederverwendung ist das sog. *"physikalische* Recycling", bei welchem Polyurethanabfälle mechanisch zerkleinert und in der Herstellung neuer Produkte eingesetzt werden. Dieser Art des Recyclings sind naturgemäß Grenzen gesetzt, weshalb es nicht an Versuchen gefehlt hat, die der Polyurethanproduktion *zugrunde liegenden Rohstoffe* durch Rückspaltung der Polyurethanbindungen wiederzugewinnen (sog. *"chemisches* Recycling").

Verfahren zum chemischen Recycling von Polyurethanen beruhen vielfach auf der Umsetzung von Urethanen mit Wasser, wobei Amine, Polyole und Kohlenstoffdioxid freigesetzt werden (Hydrolyse). Auch die Umsetzung von Polyurethanen mit Alkoholen ist beschrieben (Simon et al., 2018, "Recycling of polyurethanes from laboratory to industry, a journey towards the sustainability", Waste Management 76: 147-171). Hierbei wird das zur Synthese des Polyuethans ursprünglich verwendete Polyol durch den zugesetzten Alkohol ersetzt. Dieser Vorgang wird als Alkoholyse bezeichnet. Bei der Hydroglykolyse wir das Polyurethan mit Wasser und Alkoholen gleichzeitig umgesetzt. Hierbei laufen die oben beschriebenen Vorgänge der Hydrolyse und Alkoholyse parallel ab. Die Aminolyse basiert auf demselben Prinzip wie die Alkoholyse. Allerdings wird dem abzubauenden Polyurethan hier anstatt eines Alkohols ein Amin zugesetzt (siehe Simon et al., 2018).

Der Vorteil aller oben genannten Verfahren liegt in der Bereitstellung gut definierter Reaktionsprodukte durch selektive Spaltung der Urethanbindung. Die Reaktionsprodukte sind damit grundsätzlich gute Ausgangsstoffe für die Synthese neuer Verbindungen, insbesondere von Polymeren. Bei Versuchen zur Spaltung von Polyurethanen mit den oben beschriebenen Verfahren hat sich aber herausgestellt, dass durch Nebenreaktionen Verunreinigungen durch Nebenprodukte der Spaltung auftreten und dass die Konzentration dieser Nebenprodukte für die Weiterverwendung der Spaltprodukte zu hoch ist.

Zu diesen Nebenprodukten gehören die aus dem Polyurethan freigesetzten Amine, die an der Aminogruppe alkyliert sind, so dass vor allem sekundäre Amine, teilweise auch tertiäre Amine, vorliegen. Da nicht in jedem Fall eine Verfahrensführung möglich ist, die die Bildung derartiger sekundärer und tertiärer Amine in hinreichendem Maße unterdrückt, musste ein Verfahren zur Entfernung dieser Amine aus dem Reaktionsprodukt gefunden werden.

Aufgrund der Komplexität des Gemisches nach einem chemolytischen Verfahren ist eine quantitative Abtrennung der *N*-alkylierten Nebenkomponenten von den Zielprodukten (aromatisches Amin und Polyol) derzeit für manche Polyurethane technisch schwierig oder unmöglich. Dies betrifft insbesondere Polyurethanhartschäume.

Diese Aufgabe wird durch die in den Ansprüchen und in der nachfolgenden Beschreibung offenbarten Ausführungsformen gelöst.

In einer ersten Ausführungsform betrifft die vorliegende Erfindung ein Verfahren enthaltend die Schritte
a) der Spaltung einer Urethanbindung durch Chemolyse; und anschließend
b) der enzymatischen Spaltung der in Verfahrensschritt a) entstandenen sekundären und/oder tertiären Amine mit einem Enzym ausgewählt aus der Gruppe bestehend aus
   (i) Oxidoreduktasen, die CH-NH-Gruppen oder CH-NH2-Gruppen als Elektronendonor benutzen (E.C.-Klassen 1.4.-.- und 1.5.-.-),
   (ii) Oxidoreduktasen, die Peroxide als Elektronendonor oder -akzeptor nutzen (E.C.-Klasse 1.11.-.-),
   (iii) Oxidoreduktasen, die molekularen Sauerstoff einfügen oder reduzieren (E.C.-Klasse 1.14.-.-),
   (iv) Oxidoreduktasen mit Eisen-Schwefel-Clustern (E.C.-Klasse 1.18.-.-), und
   (v) Oxidoreduktasen, welche einen Cofaktor wie NADH oder NADPH verwenden (E.C.-Klasse 1.6.-.-)

Das erfindungsgemäße Verfahren dient dem Abbau sekundärer und/oder tertiärer Amine, die während der Chemolyse als unerwünschte Nebenprodukte auftreten können. Es stellt die Zielprodukte der Chemolyse mithin in reinerer Form zur Verfügung als bisher möglich und kann deswegen auch als Verfahren zur Entfernung unerwünschter Nebenprodukte aus einem Chemolyseprodukt verstanden werden.

Der Begriff "Chemolyse" bezeichnet den Vorgang der Spaltung einer Urethanbindung in Anwesenheit wenigstens eines Chemolysereagenz ausgewählt aus der Gruppe bestehend aus primären organischen Aminen, sekundären organischen Aminen, primären Alkoholen, sekundären Alkoholen und Wasser. Das Chemolysereagenz wird hier im stöchiometrischen Überschuss eingesetzt.

Weiterhin wird bei der Chemolyse üblicherweise ein geeigneter Katalysator verwendet. Die Chemolyse des Polyurethanschaums mit einem Alkohol, Amin oder Aminoalkohol und Wasser in Gegenwart eines Katalysators läuft bei einer Temperatur im Bereich von 130 °C bis 195 °C, bevorzugt im Bereich von 135 °C bis 190 °C, besonders bevorzugt im Bereich von 140 °C bis 190 °C und ganz besonders bevorzugt im Bereich von 145 °C bis 185 °C ab.

Als Katalysatoren für die Durchführung der Chemolyse eignen sich bevorzugt (insbesondere Alkalimetall- oder Erdalkalimetall-)Hydroxide, (insbesondere Alkalimetall- oder Erdalkalimetall-)Carboxylate (insbesondere Acetate), Zinnverbindungen (insbesondere Dibutylzinndilaurat oder Zinn(II)-octoat [= Zinn(II)-2-ethylhexanoat]), Zinkverbindungen (insbesondere Zinkacetat), (insbesondere Alkalimetall- oder Erdalkalimetall-)Carbonate, (insbesondere Alkalimetall- oder Erdalkalimetall-)Orthophosphate, (insbesondere Alkalimetall- oder Erdalkalimetall-)Monohydrogen-Orthophosphate, (insbesondere Alkalimetall- oder Erdalkalimetall-)Metaphosphate oder einer Mischung von zwei oder mehr der vorgenannten Katalysatoren. Besonders bevorzugt sind (insbesondere Alkalimetall- oder Erdalkalimetall-)Carbonate, (insbesondere Alkalimetall- oder Erdalkalimetall-)Orthophosphate, (insbesondere Alkalimetall- oder Erdalkalimetall-)Monohydrogen-Orthophosphate oder Mischung von zwei oder mehr der vorgenannten Katalysatoren. Das Massenverhältnis von Katalysator und Polyurethanprodukt liegt vorzugsweise im Bereich von 0,001 bis 0,035.

Für den Verfahrensschritt a) sind grundsätzlich alle dem Fachmann bekannten Chemolyseverfahren geeignet, da in allen Fällen sekundäre und teilweise zusätzlich oder alternativ tertiäre Amine als unerwünschte Nebenprodukte entstehen können. Ein Überblick über die relevanten Verfahren wird in Simon et al., 2018 gegeben.

Allen Varianten der Chemolyse ist gemein, dass dabei die zur Synthese des Urethans eingesetzten Polyole freigesetzt werden.

Die darüber hinaus entstehenden und erwünschten Produkte variieren abhängig von der Art der Chemolyse.

Bei der Chemolyse in Anwesenheit eines primären oder sekundären Amins, nachfolgend auch als "Aminolyse" bezeichnet, entstehen zusätzlich Kohlenstoffdioxid und ein Amin, das dem zur Synthese des Urethans verwendeten Isocyanat entspricht, d.h. formal aus der Struktur des betreffenden Isocyanats durch Substitution der Isocyanatgruppe durch eine Aminogruppe abgeleitet werden kann. Das zur Aminolyse eingesetzte primäre oder sekundäre Amin wird ebenfalls am Ende der Reaktion wieder freigesetzt. Wenn zur Synthese eines Urethans unterschiedliche Isocyanate verwendet wurden, so entsteht bei der Chemolyse ein Gemisch der diesen Isocyanaten entsprechenden Amine.

Als Chemolysereagenz bei der Aminolyse sind primäre und sekundäre organische Amine sowie Aminoalkohole einsetzbar. Die Verbindungen haben ein Molekulargewicht zwischen 40 g/mol und 400 g/mol.

In einer bevorzugten Ausführungsform der vorliegenden Erfindung wird wenigstens ein Amin oder Aminoalkohol ausgewählt aus der Gruppe bestehend aus Ethanolamin (2-Aminoethanol), N-Methylethanolamin, 3-Amino-1-propanol, 1,2-Ethylendiamin, 1,4-Diaminobutan, 1,6-Hexamethylendiamin, Dimethylamin, Diethylamin, *N*-Isopropylmethylamin, Diethylenetriamin, *N*-Methyl-1,3-diaminopropan, *N*,*N*'-Dimethyl-1,3-propanediamin, *N,N-*Dimethylhydroxylaminhydrochlorid, 2-(Ethylamino)ethanol, Dipropylamin, *N,N'-*Diethylethylenediamin, Pyrrolidin, N,N-Diethylhydroxylamin, 3-Methylamino-1-propanol, *N*,*N*,*N*'-Trimethylethylenediamin, Diethanolamin, 1,4,7-Triazacyclononan, Triethylenetetramin, *N*-Methylpentylamin, 3-(Dimethylamino)-1-propylamin, *N*-Isopropylethylenediamin, *N,N'-*Diethyl-1,3-propanediamin, Dicyandiamid, Piperidin, Sarcosin, N,N'-Bis(2-hydroxyethyl)ethylenediamin, 3-[(2-Aminoethyl)amino]-1-propanol, *N*,*N*-Diethylhydroxylamin, Piperazin, *N*-Methylethanolamin, Morpholin, Tetramethylguanidin, *N*-Methylcyclohexylamin, Isopropanolamin und Diisopropanolamin als Chemolysereagens eingesetzt. Bevorzugte Amine sind Ethanolamin und Isopropanolamin.

Bei der Chemolyse in Anwesenheit eines primären oder sekundären Alkohols, nachfolgend auch als Alkoholyse" bezeichnet, entsteht ein niedermolekulares Urethan, das formal durch Austausch des zur Synthese des Polyurethans verwendeten Polyols durch den zur Chemolyse verwendeten Alkohol abgeleitet werden kann.

Als Chemolysereagenzien bei der Alkoholyse sind ein- oder mehrwertige primäre oder sekundäre Alkohole Molekulargewicht zwischen 50 g/mol und 400 g/mol einsetzbar.

In einer bevorzugten Ausführungsform der vorliegenden Erfindung wird wenigstens ein Alkohol ausgewählt aus der Gruppe bestehend aus Methanol, Ethanol, Ethylenglykol, Diethylenglykol, Propylenglykol, Dipropylenglykol, Methylglykol, Triethylenglykol, Glycerin, 2-Methyl-1,3-Propandiol, 1,4-Butandiol, 1,5-Pentandiol, 1,6-Hexandiol und Polyethylenglykol 400 eingesetzt. Diethylenglykol und Dipropylenglykol sind besonders bevorzugt.

Bei der Chemolyse in Anwesenheit von Wasser, nachfolgend auch als "Hydrolyse" bezeichnet, hängen die Nebenprodukte davon ab, welches Polyol zur Synthese des Polyurethans verwendet wurden. Bei Verwendung von Polyetherpolyolen ist Kohlenstoffdioxid das einzige Produkt, das neben dem verwendeten Isocyanate entsprechenden Amin entsteht. Wenn das Urethan ein Polyesterpolyol enthält, so kann die Hydrolyse auch zur Spaltung des Polyesterpolyols führen. Hierbei entstehen dann mehrwertige Alkohole und mehrwertige Carbonsäuren als Nebenprodukte. Bei der Spaltung von Polycaprolactan entsteht 6-Hydroxyhexansäure als bifunktionelles Nebenprodukt.

Die oben als Produkte aufgezählten Amine, Polyole und niedermolekularen Urethane sind die erwünschten Produkte des Verfahrens ("Zielprodukte"). Sie können, ggf. nach Abtrennung aus dem Reaktionsgemisch, als solche oder nach weiteren Reaktionen wieder als Chemierohstoffe eingesetzt werden. Das ebenfalls gebildete CO₂ ist zwar kein Zielprodukt des Verfahrens, stört die weitere Verarbeitung der Zielprodukte jedoch nicht.

Es entstehen aber in variablen Mengen sekundäre Amine, die für eine weitere Verwendung in der Synthese neuer Verbindungen nicht geeignet sind, deswegen also keine Zielprodukte sind. Sie stören aber bei der weiteren Verwendung der Zielprodukte.

### Sekundäre Amine

Sekundäre und/oder Amine entstehen durch die Reaktion des Amins mit einer Urethangruppe. Diese Urethangruppe kann Bestandteil des zu spaltenden Polyurethans sein. Sie kann aber auch Bestandteil eines bei der Chemolyse entstehenden niedermolekularen Urethans sein.

Dies erfolgt über einen nukleophilen Angriff der Amin- oder Hydroxylgruppe des jeweils eingesetzten Chemolysereagenz an das aromatische Carbamat. Unter Kohlenstoffdioxidabspaltung bildet sich eine sekundäre oder tertiäre Aminstruktur aus. Ob ein sekundäres oder tertiäres Amin entsteht, hängt hierbei vom verwendeten Chemolysereagenz ab. Bei der Verwendung sekundärer Amine als Chemolysereagenzien entstehen tertäre Amine als unerwünschte Nebenprodukte. Bei der Verwendung aller anderen Chemolysereagenzien sekundäre Amine.

Sekundäre Amine werden durch Formel (I) definiert: R₁-NH-R₂.

Hierbei ist R₁ abgeleitet von einem Amin ausgewählt aus der Gruppe bestehend aus Toluylen-2,4-diamin, Toluylen-2,6-diamin, Diphenylmethan-2,2'-diamin (2,2'-MDA), Diphenylmethan-2,4'-diamin (2,4'-MDA), Diphenylmethan-4,4'-diamin (4,4'-MDA), polymerem 2,2'-MDA, 2,4'-MDA und 4,4'-MDA und Naphthylen-1,5-diamin. "Abgeleitet" bedeutet dabei, dass die in der allgemeinen Formel gezeigte sekundäre Aminogruppe formal durch Substitution eines Wasserstoffatom einer der primären Aminogruppen des besagten Amins durch den Rest R₂ erhalten wird.

R₂ ist hierbei ein Alkylrest abgeleitet von
(i) einem Alkohol ausgewählt aus der Gruppe bestehend aus Methanol, Ethanol, Ethylenglykol, Diethylenglykol, Propylenglykol, Dipropylenglykol, Methylglykol, Triethylenglykol, Glycerin, 2-Methyl-1,3-Propandiol, 1,4-Butandiol, 1,5-Pentandiol, 1,6-Hexandiol und Polyethylenglykol 400; oder
(ii) einem Aminoalkohol ausgewählt aus der Gruppe bestehend aus Ethanolamin (2-Aminoethanol), *N*-Methylethanolamin, 3-Amino-1-propanol, *N- N,N-*Dimethylhydroxylaminhydrochlorid, 2-(Ethylamino)ethanol, N,N-Diethylhydroxylamin, 3-Methylamino-1-propanol, Diethanolamin, N,N'-Bis(2-hydroxyethyl)ethylenediamin, 3-[(2-Aminoethyl)amino]-1-propanol, *N,N-*Diethylhydroxylamin, *N*-Methylethanolamin; oder
(iii) einem Amin ausgewählt aus der Gruppe bestehend aus 1,2-Ethylendiamin, 1,4-Diaminobutan, 1,6-Hexamethylendiamin, Dimethylamin, Diethylamin, Isopropylmethylamin, Diethylenetriamin, *N*-Methyl-1,3-diaminopropan, *N,N'-*Dimethyl-1,3-propanediamin, Dipropylamin, *N*,*N*'-Diethylethylenediamin, *N*,*N,N*'-Trimethylethylenediamin, Triethylenetetramin, *N*-Methylpentylamin, 3-(Dimethylamino)-1-propylamin, *N*-Isopropylethylenediamin, *N*,*N*'-Diethyl-1,3-propanediamin, Dicyandiamid, Sarcosin, *N*-Methylcyclohexylamin, Isopropanolamin und Diisopropanolamin und Tetramethylguanidin.

R₂ wird formal von einem Alkohol (i) oder Aminoalkohol (ii) dadurch abgeleitet, dass eine Hydroxylgruppe des Alkohols oder Aminoalkohols durch das Stickstoffatom der allgemeinen Formel substituiert wird. Hier ist das Stickstoffatom formal Bestandteil des Restes R₁. Von einem der unter (iii) genannten primären Amine wird R₂ dadurch abgeleitet, dass eine Aminogruppe des Restes R₁ durch eine primäre oder sekundäre Aminogruppe eines der in (iii) genannten Amine ausgetauscht wird. Hier ist das Stickstoffatom in der allgemeinen Formel formal Bestandteil des primären Amins aus Gruppe (iii).

Tertiäre Amine werden durch Formel (II) definiert: R₁-R₂.

R₁ entspricht hierbei einem Radikal, das von einem Amin ausgewählt aus der Gruppe bestehend aus Toluylen-2,4-diamin, Toluylen-2,6-diamin, Diphenylmethan-2,2'-diamin (2,2'-MDA), Diphenylmethan-2,4'-diamin (2,4'-MDA), Diphenylmethan-4,4'-diamin (4,4'-MDA), polymerem 2,2'-MDA, 2,4'-MDA und 4,4'-MDA und Naphthylen-1,5-diamin formal durch Entfernung einer Aminogruppe abgeleitet ist.

R₂ wird von einem sekundären Amin ausgewählt aus der Gruppe bestehend aus Pyrrolidin, 1,4,7-Triazacyclononan, Piperidin, Piperazin und Morpholin dadurch abgeleitet, dass formal das Wasserstoffstoffatom an einer sekundären Aminogruppe eines der vorgenannten Amine durch das von R₁ abgeleitete Radikal ersetzt wird.

Der Fachmann versteht, dass bei der Verwendung eines Gemisches unterschiedlicher Chemolysereagenzien Gemische von sekundären Aminen entstehen, die sich durch R₂ unterscheiden. R₂ ist in diesen Fällen von mehreren der oben genannten Verbindungen abgeleitet. Dies gilt auch für die Alkoholyse oder Aminolyse von Polyesterpolyolen, da hier sowohl der aus dem Polyesterpolyol freigesetzte Alkohol als auch das Chemolyseragenz das freigesetzte Amin alkylieren kann.

### Urethanbindung

Eine Urethanbindung entsteht durch die Reaktion einer Hydroxylgruppe mit einer Isocyanatgruppe. Zur Synthese von Polymeren, d.h. Polyurethanen, werden mehrwertige Alkohole ("Polyole") und Isocyanate mit mehr als einer Isocyanatgruppe pro Molekül verwendet. Insbesondere werden Polyole mit wenigstens zwei Hydroxylgruppen und Isocyanate mit wenigstens zwei Isocyanatgruppen pro Molekül verwendet.

Die erfindungsgemäß einzusetzenden Urethane basieren vorzugsweise auf wenigstens einem Isocyanat ausgewählt aus der Gruppe bestehend aus Toluylen-2,4-diisocyanat, Toluylen-2,6-diisocyanat, Diphenylmethan-2,2'-diisocyanat (2,2'-MDI), Diphenylmethan-2,4'-diisocyanat (2,4'-MDI), Diphenylmethan-4,4'-diisocyanat (4,4'-MDI), polymerem 2,2'-MDI, 2,4'-MDI und 4,4'-MDI und Naphthylen-1,5-diisocyanat.

Im Sinne der vorliegenden Erfindung werden unter *"Di- und Polyisocyanaten der Diphenylmethanreihe"* Isocyanate und Gemische von Isocyanaten folgenden Typs verstanden:

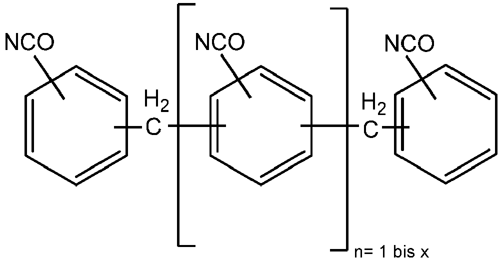

Dabei steht x für eine natürliche Zahl ≥ 1. Im Folgenden werden die Verbindungen dieses Typs, bei denen x = 1 ist, auch als Diisocyanate der Diphenylmethanreihe oder Diisocyanatdiphenylmethane (nachfolgend MMDI) bezeichnet. Verbindungen dieses Typs, bei denen x > 1 ist, werden im Rahmen dieser Erfindung auch als Polyisocyanate der Diphenylmethanreihe oder Polyphenylenpolymethylenpolyisocyanate (nachfolgend PMDI) bezeichnet. Gemische aus beiden Typen werden auch als Di- und Polyisocyanate der Diphenylmethanreihe bezeichnet (nachfolgend MDI). Industriell werden die Di- und Polyamingemische überwiegend durch Phosgenierung zu den entsprechenden Di- und Polyisocyanaten der Diphenylmethanreihe umgesetzt.

Bevorzugt sind Urethane, insbesondere Polyurethane, bei deren Synthese die oben beschriebenen Isomere des MDI oder des polymeren MDI verwendet wurden.

Das zur Synthese der erfindungsgemäß einzusetzenden Urethane verwendete Polyol ist ausgewählt aus der Gruppe bestehend aus Polyetherpolyolen, Polyesterpolyolen Polyetheresterpolyolen, Polycarbonatpolyolen, Polyetherpolycarbonatdiolen und Polyesterpolycarbonatdiolen. Wenn es sich der Chemolyse in Verfahrensschritt a) um eine Hydrolyse handelt, ist das Polyol vorzugsweise ein Polyesterpolyol.

Polyurethanhartschäume werden im Allgemeinen unter Verwendung vergleichsweise kurzkettiger Polyole, insbesondere kurzkettiger Polyetherpolyole, hergestellt. Die kurzkettigen Polyetherpolyole basieren vorzugsweise auf Zuckerstartern (wie zum Beispiel Saccharose oder Sorbit) insbesondere mit Abmischungen von Glykolen (wie zum Beispiel Ethylenglykol oder Propylenglykol) oder aromatischen Aminen (wie zum Beispiel Toluylendiamin, insbesondere das 2,4-Isomer) als weiteren Startern. Sie weisen eine (theoretische, d. h. dem verwendeten Starter entsprechende) Hydroxyfunktionalität von 2 bis 8, bevorzugt 2 bis 6, auf, sowie eine zahlengemittelte Molmasse Mn im Bereich von 400 g/mol bis 1500 g/mol, bevorzugt 400 g/mol bis 1000 g/mol. Die Bestimmung von Molmassen erfolgt im Rahmen der vorliegenden Erfindung durch Gelpermeationschromatographie (GPC). Zur Bestimmung der massengemittelten Molmasse Mw, der zahlenmittleren Molmasse Mn sowie der Polydispersität Mw/Mn wurden folgende Messbedingungen eingehalten:
Säulenkombination SDV 1000/10000 Å (Länge 65 cm), Temperatur 30 °C, THF als mobile Phase, Fließrate 1 ml/min, Probenkonzentration 10 g/l, RI-Detektor. Die Auswertung der Polyetherpolyole erfolgte gegen Polystyrol-Standard (162 g/mol bis 2.57 × 104 g/mol). Die OH-Zahlen solcher Polyetherpolyole liegen im Bereich von 100 mgKOH/g bis 600 mgKOH/g.

Werden Mischungen verschiedener Polyetherpolyole eingesetzt, so gelten die vorgenannten Werte für die Molmasse und die Hydroxyfunktionalität für jedes Polyetherpolyol, das Bestandteil der Mischung ist, sodass auch die über alle Polyetherpolyole der Mischung gemittelten Werte in den genannten Bereichen liegen.

### Enzyme

Für die Verwendung in Verfahrensschritt b) sind grundsätzlich alle Oxidoreduktasen geeignet ausgewählt aus der Gruppe bestehend aus
(i) Oxidoreduktasen, die CH-NH-Gruppen oder CH-NH2-Gruppen als Elektronendonor benutzen (E.C.-Klassen 1.4.-.- und 1.5.-.-),
(ii) Oxidoreduktasen, die Peroxide als Elektronendonor oder -akzeptor nutzen (E.C.-Klasse 1.11.-.-),
(iii) Oxidoreduktasen, die molekularen Sauerstoff einfügen oder reduzieren (E.C.-Klasse 1.14.-.-),
(iv) Oxidoreduktasen mit Eisen-Schwefel-Clustern (E.C.-Klasse 1.18.-.-), und
(v) Oxidoreduktasen, welche einen Cofaktor wie NADH oder NADPH verwenden (E.C.-Klasse 1.6.-.-)

Hierbei sind die Oxidoreduktasen der Gruppen (i), (ii) und (iii) stärker und Oxidoreduktasen der Gruppen (i) und (ii) besonders bevorzugt.

Bevorzugte Oxidoreduktasen der Klasse 1.4.-.- sind Enzyme der E.C-Klassen 1.4.3.4 (Monoaminooxidasen) und 1.4.3.12 (Cyclohexaminoxidasen) sowie Kupfer-Aminoxidasen (E.C.-Klasse 1.4.3.6) und L-Aminosäureoxidasen (E.C.-Klasse 1.3.3.2), insbesondere die Enzyme der E.-C.-Klassen 1.4.3.4 und 1.4.3.12

In der E.C.-Klasse 1.5.-.- sind Enzyme der E.C.-Klasse 1.5.1.48 (Iminreduktase) bevorzugt.

In der E.C.-Klasse 1.11.-.- sind Peroxygenasen (E.C.-Klasse 1.11.2.1) und Haloperoxidasen (E.C.-Klassen 1.11.1.10 und 1.11.1.18 bevorzugt), insbesondere solche der E.C.-Klasse 1.11.2.1.

In der E.C.-Klasse 1.14.-.- sind Cytochrom-P450-Oxidasen in der E.C.-Klasse 1.14.-.-, und α-Ketoglutarat abhängige Oxygenasen der E.C.-Klasse 1.14.11 bevorzugt.

Hierbei werden solche Oxidoreduktasen bevorzugt, die in Lage sind, wenigstens ein Modellsubstrat ausgewählt aus der Gruppe bestehend 4,4'-Methylenbis(*N*-methylanilin), 4,4'-Methylenbis(*N*,*N*-dimethylanilin), 4,4'-Methylenbis(*N*-ethylanilin), *N-*Ethylanilin, *N*-Benzylanilin und *N*-Ethyl-*N*-methylanilin unter den in den Ausführungsbeispielen beschriebenen Bedingungen zu spalten. Besonders bevorzugt ist das Modellsubstrat ausgewählt aus der Gruppe bestehend aus 4,4'-Methylenbis(*N*-methylanilin), 4,4'-Methylenbis(/V,/V-dimethylanilin), 4,4'-Methylenbis(*N*-ethylanilin).

### Verwendung

In einer weiteren Ausführungsform betrifft die vorliegende Erfindung die Verwendung eines Enzyms ausgewählt aus der Gruppe bestehend aus
(i) Oxidoreduktasen, die CH-NH-Gruppen oder CH-NH2-Gruppen als Elektronendonor benutzen (E.C.-Klassen 1.4.-.- und 1.5.-.-),
(ii) Oxidoreduktasen, die Peroxide als Elektronendonor oder -akzeptor nutzen (E.C.-Klasse 1.11.-.-),
(iii) Oxidoreduktasen, die molekularen Sauerstoff einfügen oder reduzieren (E.C.-Klasse 1.14.-.-),
(iv) Oxidoreduktasen mit Eisen-Schwefel-Clustern (E.C.-Klasse 1.18.-.-), und
(v) Oxidoreduktasen, welche einen Cofaktor wie NADH oder NADPH verwenden (E.C.-Klasse 1.6.-.-)

zur Spaltung sekundärer und/oder tertiärer Amine mit der allgemeinen Formel (I) oder (II).

Die nachfolgenden Ausführungsbeispiele dienen nur dazu, die vorliegende Erfindung zu illustrieren. Sie sollen den Schutzbereich der Patentansprüche in keiner Weise beschränken.

### Ausführungsbeispiele

### Vorgeschlagene Mechanismen zur enzymatischen Spaltung von N-Alkylverbindungen

Zur Oxidation von *N*-Alkylierten Verbindungen wurden insbesondere Monoamine/Cycloamine Oxidasen (im folgenden Verlauf MAOs oder CAOs genannt, E.C. 1.4.3.4 und E.C.1.4.3.12) und Unspezifische Peroxidasen (im folgenden Verlauf UPOs genannt, E.C. 1.11.2.1) experimentell analysiert. In beiden Fällen soll eine Oxidation an der Stickstoff-Kohlenstoff-Bindung oder an einem der beiden Atome erfolgen.

Bei MAO/CAOs dient molekularer Sauerstoff als Oxidationsmittel, aus dem dann enzymatisch H₂O₂ gebildet wird. Bei UPOs wird H₂O₂ direkt eingesetzt.

### Hintergründe zu den ausgewählten Enzymen aus dem Ausführungsbeispiel und Enzym-Präparation

Die in den Ausführungsbeispielen verwendeten Enzyme sind in Tabelle 1 zusammengefasst.

Die vier Enzyme UPO #13, UPO #13M1, UPO #13M3 und UPO #13M5 wurden von Aminoverse B.V. bereitgestellt. Zusätzlich wurde das Wildtyp AaeUPO aus der Basidiomycete *Agrocybe aegerita* (Ullrich, R., et al. (2004). "Novel haloperoxidase from the agaric basidiomycete Agrocybe aegerita oxidizes aryl alcohols and aldehydes." Appl Environ Microbiol 70(8): 4575-4581) von Aminoverse B.V. erworben.

Die Expression der in Tabelle 1 gezeigten MAOs und CAOs erfolgte im Stamm *E. coli* BL21(DE3). Der Zellaufschluss erfolgte durch Ultraschallbehandlung und die Enzymreinigung über einen N-terminalen His-tag und das IMAC-System (Nickel-NTA) nach Angaben des Herstellers (Fisher Scientific GmbH) mit anschließender Umpufferung in einen Lagerpuffer (25 mM Tris/HCl pH 7.8, 1 mM dithiothreitol (DTT), 1 mM PMSF, 300 mM NaCl) über PD-10-Säulen nach Angaben des Herstellers (cytiva). Die erfolgreiche Expression und Aufreinigung wurde über ein SDS-PAGE kontrolliert.

**Tabelle 1: Untersuchte Enzyme im Rahmen der enzymatischen N-Dealkylierung.**

| **Enzym** | **Mikrobieller Ursprung** | **Bezugsquelle** | **GenBank** | **SEQ ID** | **Expressions-system** |
|---|---|---|---|---|---|
| | ***MAOs*/*CAOs*** | | | | |
| MaoN | *Aspergillus niger* | | AAA98490.1 | SEQ ID #1 | *Escherichia coli* BL21(DE3) pET21a |
| MaoN5 Asn336Ser / Met348Lys / Ile246Met / Thr384Asn / Asp385Ser | *Aspergillus niger* | | (siehe MaoN) | SEQ ID #2 | *Escherichia coli* BL21(DE3) pET21a |
| ChaoA L225A | *Brevibacterium oxydans* strain IH-35A | | BAN13413.1 | SEQ ID #3 | *Escherichia coli* BL21(DE3) pET21a |
| ChaoA M226T Y321I | *Brevibacterium oxydans* strain IH-35A | | (siehe ChaoA L225A) | SEQ ID #4 | *Escherichia coli* BL21(DE3) pET21a |
| ChaO YT02 | *Acinetobacter* sp. YT-02 | | PCN59881 | SEQ ID #5 | *Escherichia coli* BL21(DE3) pET21a |

| | ***UPOs*** | | | | |
|---|---|---|---|---|---|
| UPO #13 | Unknown | Aminoverse B.V. | - | - | *Komagataella phaffii* (*Pichia pastoris*) strain BSYBG11JP-HP |
| UPO #13M1 | Unknown | Aminoverse B.V. | - | - | |
| UPO #13M5 | Unknown | Aminoverse B.V. | - | - | |
| AaeUPO | *Agrocybe aegerita* | | FM872457.1 | SEQ ID #6 | |

### Modellsubstrate

Die im Rahmen dieser Anwendungsbeispiele gezeigten Substrate sind in Tabelle 2 zusammengefasst. Für die Modelle *N*,*N*'-Dimethyl-4,4'-MDA und *N*,*N*'-Diethyl-4,4'-MDA wurden je *N*-Methylanilin und *N*-Ethylanilin mit Formalin umgesetzt und aufgearbeitet. Unter anderem wurden destillativ die Ausgangssubstrate entfernt und die 2-Kern-Derivate angereichert. Zusätzlich wurde eine Probe angereichert mit *N*-Methyl-4,4'-MDA als Standard verwendet, die eine qualitative - aber keine quantitative - Bestimmung zuließ.

**Tabelle 2: Untersuchte Modellsubstrate und Bezugsquellen.**

| **Modell** | **Struktur** | **Bezugsquelle** |
|---|---|---|
| *N*-Ethylanilin | 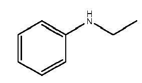 | Sigma-Aldrich |
| *N*-Benzylanilin | 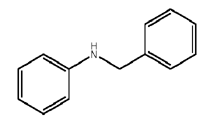 | Sigma-Aldrich |
| *N*-Ethyl-*N*-Methylanilin³ | 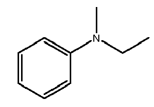 | Merck |
| *N*,*N*,*N*',*N'*-Tetramethyl-4,4'-MDA (*N*,*N*,*N*',*N*'-Tetramethyl-4,4'-diaminodiphenylmethan) | 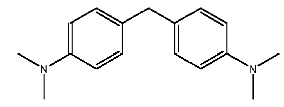 | Merck |
| *N*,*N*'-Dimethyl-4,4'-MDA | 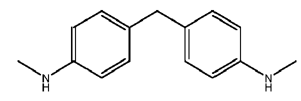 | Selbst synthetisiert |
| *N*,*N*'-Diethyl-4,4'-MDA | 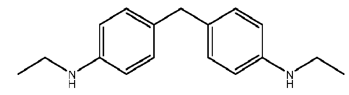 | Selbst synthetisiert |

### Enzymassays und Analytik

Für MAOs/CAOs erfolgte die Reaktion im 200 µL Maßstab bei 37 °C für mindestens 48 h und 200 rpm. Dazu wurden 100 µL der oben beschriebenen Enzympräparate in Phosphatpuffer (bevorzugt zwischen 50 bis 200 mM, pH 7,5) mit 10 mM eines der sechs in Tabelle 2 gelisteten Substrate versetzt. Als Co-Solvenz für das Substrat wurde Dimethylsulfoxid eingesetzt (maximal 5 % (w/w) der finalen Umsetzungsreaktion). Um eine ausreichende Sauerstoffversorgung über den Kopfraum des Reaktionsansatzes zu gewährleisten, wurden Reaktionsgefäße oder Mikrotiterplatten in eckiger Ausführungsform und 10-fachem Gesamtvolumen bevorzugt.

Für UPOs erfolgte die Reaktion im 300 µL Maßstab in Tricine Puffer (100 mM) bei 30 °C für mindestens 4 h und 200 rpm. Dazu wurden die von Aminoverse B.V. bereitgestellten Enzympräparate in dH₂O gelöst (ca. 0,575 mg/mL) und 30 µL der Enzymlösung in den Assay eingesetzt, welcher mit 4 mM eines der sechs in Tabelle 2 gelisteten Substrate versetzt war. Der Assay wurde mit Zugabe des Co-Substrats H₂O₂ (1,5 µL einer 200 mM H₂O₂-Lösung) gestartet, welches alle 30 min wiederholt zugegeben wurde (final 12 µL H₂O₂).

Als Negativkontrollen wurden für alle Substrate und Bedingungen Assays ohne Enzymlösung durchgeführt. Alle Assays erfolgten mindestens in Triplikaten.

Zum Abstoppen der enzymatischen Reaktion und Extraktion erfolgte die Verdünnung des Reaktionsansatzes in gleichen Teilen Acetonitril. Die Proben wurden filtriert und die Produktfreisetzung per HPLC analysiert (ZORBAX Eclipse C18 Säule (Partikelgröße von 3,5 pm, 4,6 × 75 mm (Agilent Technologies, Santa Clara, USA), 40 °C, Laufmittel A: Acetonitril mit 5 % Reinstwasser, Laufmittel B: 10 mM Natriumphosphat-Puffer pH 7,0 mit 5 % ACN, Fluss: 1 mL/min). Das Profil der HPLC-Methode ist in Tabelle 3 gezeigt. Tabelle 4 zeigt die Elutionszeiten der Komponenten mit dieser Messmethode.

**Tabelle 3: Profil der HPLC-Methode.**

| **Zeit / min** | **A %** | **B%** | **Fluss / ml · min⁻¹** | **Maximal zulässiger Druck / bar** |
|---|---|---|---|---|
| 0,00 | 0 | 100 | 1 | 300 |
| 2,00 | 0 | 100 | 1 | 300 |
| 10,00 | 95 | 5 | 1 | 300 |
| 11,00 | 95 | 5 | 1 | 300 |
| 11,50 | 0 | 100 | 1 | 300 |
| 17,00 | 0 | 100 | 1 | 300 |

**Tabelle 4: Elutionszeiten der Modellsubstrate und Zielprodukte bei der in diesem Ausführungsbeispiel verwendeten HPLC-Analytik.**

| **Komponente** | **Elutionszeit (min)** | **Genutzte Wellenlänge** |
|---|---|---|
| Anilin | 7,4 | 235 nm |
| 4,4'-MDA | 8,4 | 254 nm |
| *N*-Methyl-4,4'-MDA | 9,5 | 254 nm |
| *N*-Ethylanilin | 10,0 | 254 nm |
| *N*,*N*'-Dimethyl-4,4'-MDA | 10,5 | 254 nm |
| *N*-Benzylanilin | 10,9 | 254 nm |
| *N*-Ethyl-*N*-Methylanilin | 11,0 | 254 nm |
| *N*,*N*'-Diethyl-4,4'-MDA | 11,4 | 254 nm |
| *N*,*N*,*N*',*N*'-Tetramethyl-4,4'-MDA | 11,2 | 254 nm |

### Ergebnisse

Sowohl MAOs/CAOs als auch UPOs zeigten Aktivität an *N*-alkylierten Verbindungen; sie unterschieden sich dabei in ihrer Spezifität und Selektivität (Tabelle 5) sowie Aktivität.

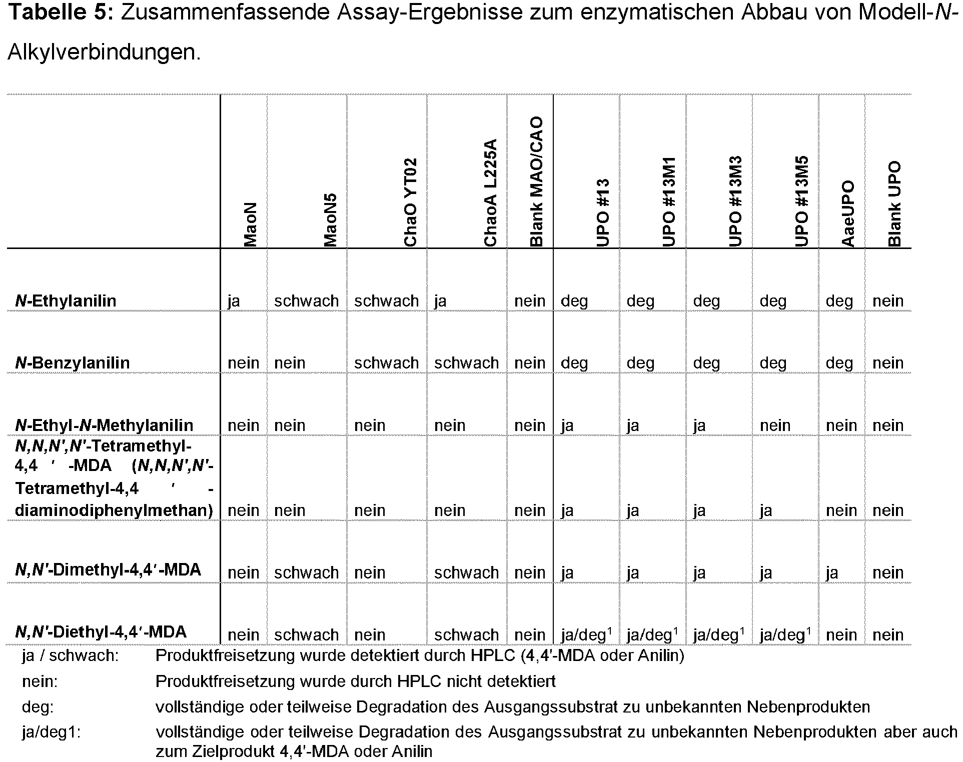

### Anilin-basierte N-alkylierte Substrate

Für die Anilin-basierten Substrate *N*-Ethylanilin und *N*-Benzylanilin, zeigten MAOs/CAOs spezifische Aktivität, die zur gezielten Anilin-Freisetzung führten (bei *N*-Benzylanilin nur: ChaO YT02 und ChaoA L225A). Bei allen UPOs inklusive der Wildtyp-Variante AaeUPO, wurden *N-*Ethylanilin und *N*-Benzylanilin vollständig zu unbekannten Nebenprodukten degradiert, was auf Überoxidation des Substrats hindeutet.

Für das tertiäre Amin *N*-Ethyl-*N*-Methylanilin konnte bei Behandlung mit MAOs/CAOs keine Aktivität festgestellt werden, während bei UPO #13, UPO #13M1, UPO #13M3 Anilin als Zielprodukte nachgewiesen werden konnte.

### 4,4'-MDA-basierte N-alkylierte Substrate

Die N-methylierte Verbindung *N*,*N*'-Dimethyl-4,4'-MDA wurde von den MAOs/CAOs MaoN5 und ChaoA L225A zu 4,4'-MDA umgesetzt, wenn auch mit geringerer Aktivität. In den UPO-Assays wurde dieses Substrat durch die Enzyme UPO #13, #13M3 und #13M5 vollständig abgebaut, so dass dieses nicht mehr in den HPLC-Chromatogrammen detektiert werden konnte. Auch bei UPO #13M1 und AaeUPO zeigt sich ein starker Abbau (78 mol% bzw. 62 mol% des eingesetzten Substrates wurden abgebaut). In der Negativkontrolle wurde das Ausgangssubstrat vollständig wiedergefunden. Des Weiteren wurde das Zielprodukt 4,4`-MDA in UPO-Assays spezifisch freigesetzt, allerdings nicht in den gleichen molaren Mengen (mol% bezogen auf das eingesetzte Substrat, AaeUPO: 0,7 mol%, UPO #13: 2,1 mol%, UPO #13M1: 2,5 mol%, UPO #13M3: 2,8 mol% und UPO #13M5: 2,2 mol%). Dies ist dadurch zu erklären, dass bis zum Zielprodukt zwei Dealkylierungen stattfinden müssen, da es sich bei dem Ausgangssubstrat um ein Diamin handelt. Entsprechend wurde ein mögliches gebildetes Zwischenprodukt durch die Bildung eines Peaks nach 9,5 min angezeigt, welches einfach methyliertes 4,4'-MDA (*N*-Methyl-4,4'-MDA) darstellt. Weitere (unspezifische) Nebenprodukte wurden nicht detektiert.

Entsprechend konnte für das (komplexere) tertiäre Amin *N*,*N*,*N*'*N*'-Tetramethyl-4,4'-MDA (*N*,*N*,*N*',*N*'-Tetramethyl-4,4'-diaminodiphenylmethan) bei Behandlung mit MAOs/CAOs keine reproduzierbare Aktivität festgestellt werden, während für alle UPOs 4,4'-MDA als Zielprodukte nachgewiesen werden konnte (mol% bezogen auf das eingesetzte Substrat, AaeUPO: 0,1 mol%, UPO #13: 1,1 mol%, UPO #13M1: 1,1 mol%, UPO #13M3: 1,4 mol% und UPO #13M5: 1,3 mol%). Auch *N*,*N*'-Dimethyl-4,4'-MDA - hier Zwischenprodukt der enzymatischen Reaktion -wurde nachgewiesen (mol% bezogen auf das eingesetzte Substrat, AaeUPO: 0 mol%, UPO #13: 25,7 mol%, UPO #13M1: 31,8 mol%, UPO #13M3: 27,5 mol% und UPO #13M5: 27,9 mol%), sowie 2 weitere Peaks, die auf weitere Zwischenprodukte hindeuten (9,5 min: einfach methyliertes Zwischenprodukt *N*-Methyl-4,4'-MDA; 11,4 min: mögliches dreifach methyliertes Zwischenprodukt).

Für das *N*-ethylierte Substrat *N*,*N*'-Diethyl-4,4'-MDA zeigte sich bei allen UPOs außer AaeUPO ein breites Spektrum an Nebenkomponenten, jedoch wurde auch 4,4'-MDA-Freisetzung detektiert (mol% bezogen auf das eingesetzte Substrat, AaeUPO: 0 mol%, UPO #13: 2,6 mol%, UPO #13M1: 2,5 mol%, UPO #13M3: 2,9 mol% und UPO #13M5: 2,8 mol%). Die MAOs/CAOs MaoN5 und ChaoA L225A zeigten ähnlich wie beim *N*-methylierten Amin nur sehr schwache 4,4'-MDA-Freisetzung.

## Patentansprüche

1. Verfahren enthaltend die Schritte
a) der Spaltung einer Urethanbindung durch Chemolyse; und anschließend
b) der enzymatischen Spaltung der in Verfahrensschritt a) entstandenen sekundären und/oder tertiären Amine mit einem Enzym ausgewählt aus der Gruppe bestehend aus
(i) Oxidoreduktasen der E.C.-Klassen 1.4.-.- und 1.5.-.-, die CH-NH-Gruppen oder CH-NH₂-Gruppen als Elektronendonor benutzen ,
(ii) Oxidoreduktasen der E.C.-Klasse 1.11.-.-, die Peroxide als Elektronendonor oder -akzeptor nutzen,
(iii) Oxidoreduktasen der E.C.-Klasse 1.14.-.-, die molekularen Sauerstoff einfügen oder reduzieren,
(iv) Oxidoreduktasen der E.C.-Klasse 1.18.-.- mit Eisen-Schwefel-Clustern, und
(v) Oxidoreduktasen der E.C.-Klasse 1.6.-.-, welche einen Cofaktor wie NADH oder NADPH verwenden.

2. Das Verfahren nach Anspruch 1, wobei die Chemolyse unter Verwendung eines Chemolysereagenz ausgewählt aus der Gruppe bestehend aus Aminen mit einem Molekulargewicht zwischen 40 g/mol und 400 g/mol, Alkoholen mit einem Molekulargewicht zwischen 50 g/mol und 400 g/mol und Aminoalkoholen mit einem Molekulargewicht zwischen 40 g/mol und 400 g/mol durchgeführt wird.

3. Das Verfahren nach Anspruch 1 oder 2, wobei das sekundäre Amin durch die allgemeine Formel (I) R₁-NH-R₂ definiert ist und wobei
R₁ abgeleitet ist von einem Amin ausgewählt aus der Gruppe bestehend aus Toluylen-2,4-diamin, Toluylen-2,6-diamin, Diphenylmethan-2,2'-diamin (2,2'-MDA), Diphenylmethan-2,4'-diamin (2,4'-MDA), Diphenylmethan-4,4'-diamin (4,4'-MDA), polymerem 2,2'-MDA, 2,4'-MDA und 4,4'-MDA und Naphthylen-1,5-diamin; und
R₂ ein Alkylrest ist abgeleitet von
(i) einem Alkohol ausgewählt aus der Gruppe bestehend aus Methanol, Ethanol, Ethylenglykol, Diethylenglykol, Propylenglykol, Dipropylenglykol, Methylglykol, Triethylenglykol, Glycerin, 2-Methyl-1,3-Propandiol, 1,4-Butandiol, 1,5-Pentandiol, 1,6-Hexandiol und Polyethylenglykol 400; oder
(ii) einem Aminoalkohol ausgewählt aus der Gruppe bestehend aus Ethanolamin (2-Aminoethanol), N-Methylethanolamin, 3-Amino-1-propanol, *N- N,N-*Dimethylhydroxylaminhydrochlorid, 2-(Ethylamino)ethanol, N,N-Diethylhydroxylamin, 3-Methylamino-1-propanol, Diethanolamin, N,N'-Bis(2-hydroxyethyl)ethylenediamin, 3-[(2-Aminoethyl)amino]-1-propanol, *N,N-*Diethylhydroxylamin, *N*-Methylethanolamin; oder
(iii) einem Amin ausgewählt aus der Gruppe bestehend aus 1,2-Ethylendiamin, 1,4-Diaminobutan, 1,6-Hexamethylendiamin, Dimethylamin, Diethylamin, Isopropylmethylamin, Diethylenetriamin, *N*-Methyl-1,3-diaminopropan, *N,N'-*Dimethyl-1,3-propanediamin, Dipropylamin, *N*,*N*'-Diethylethylenediamin, *N*,*N*,*N*'-Trimethylethylenediamin, Triethylenetetramin, *N*-Methylpentylamin, 3-(Dimethylamino)-1-propylamin, *N*-Isopropylethylenediamin, *N*,*N*'-Diethyl-1,3-propanediamin, Dicyandiamid, Sarcosin, N-Methylcyclohexylamin, Isopropanolamin und Diisopropanolamin und Tetramethylguanidin.

4. Das Verfahren nach Anspruch 1 oder 2, wobei das tertiäre Amin durch die allgemeine Formel (II) definiert ist und wobei
R₁ einem Radikal entspricht, das von einem Amin ausgewählt aus der Gruppe bestehend aus Toluylen-2,4-diamin, Toluylen-2,6-diamin, Diphenylmethan-2,2'-diamin (2,2'-MDA), Diphenylmethan-2,4'-diamin (2,4'-MDA), Diphenylmethan-4,4'-diamin (4,4'-MDA), polymerem 2,2'-MDA, 2,4'-MDA und 4,4'-MDA und Naphthylen-1,5-diamin formal durch Entfernung einer Aminogruppe abgeleitet ist; und
R₂ von einem sekundären Amin ausgewählt aus der Gruppe bestehend aus Pyrrolidin, 1,4,7-Triazacyclononan, Piperidin, Piperazin und Morpholin dadurch abgeleitet ist, dass formal das Wasserstoffstoffatom an einer sekundären Aminogruppe eines der vorgenannten Amine durch das von R₁ abgeleitete Radikal ersetzt wird.

5. Das Verfahren nach einem der Ansprüche 1 bis 4, wobei die in Verfahrensschritt a) gespaltene Urethanbindung durch Reaktion eines Isocyanats ausgewählt aus der Gruppe bestehen aus Toluylen-2,4-diisocyanat, Toluylen-2,6-diisocyanat, Diphenylmethan-2,2'-diisocyanat (2,2'-MDI), Diphenylmethan-2,4'-diisocyanat (2,4'-MDI), Diphenylmethan-4,4'-diisocyanat (4,4'-MDI), polymerem 2,2'-MDI, 2,4'-MDI und 4,4'-MDI und Naphthylen-1,5-diisocyanat mit einem mehrwertigen Alkohol entsteht.

6. Das Verfahren nach Anspruch 5, wobei der mehrwertige Alkohol ausgewählt ist aus der Gruppe bestehend aus Polyetherpolyolen, Polyesterpolyolen Polyetheresterpolyolen, Polycarbonatpolyolen, Polyetherpolycarbonatdiolen und Polyesterpolycarbonatdiolen.

7. Das Verfahren nach einem der Ansprüche 1 bis 6, wobei
(i) Die Oxidoreduktase der E.C.-Klasse 1.4.-.- ein Enzym der E.C-Klassen 1.4.3.4 (Monoaminooxidasen), 1.4.3.12 (Cyclohexaminoxidasen), 1.4.3.6 (Kupfer-Aminoxidasen) oder 1.3.3.2 (L-Aminosäureoxidasen) ist;
(ii) Die Oxidoreduktase der E.C.-Klasse 1.5.-.- ein Enzym der E.C-Klasse 1.5.1.48 (Iminreduktase) ist;
(iii) Die Oxidoreduktase der E.C.-Klasse 1.11.-.- ein Enzym der E.C.-Klasse, 1.11.2.1 (Peroxygenasen), 1.11.1.10 oder 1.11.1.18 (Haloperoxidasen) ist; und
(iv) Oxidoreduktase der E.C.-Klasse 1.14.-.- eine Cytochrom-P450-Oxidasen der E.C.-Klasse 1.14.-.- oder eineα-Ketoglutarat abhängige Oxygenasen der E.C.-Klasse 1.14.11 ist.

8. Das Verfahren nach einem der Ansprüche 1 bis 7, wobei die Oxidoreduktase in der Lage ist, wenigstens ein Substrat ausgewählt aus der Gruppe bestehend aus 4,4'-Methylenbis(*N*-methylanilin), 4,4'-Methylenbis(/V,/V-dimethylanilin), 4,4'-Methylenbis(*N-*ethylanilin), *N*-Ethylanilin, *N*-Benzylanilin und *N*-Ethyl-*N*-methylanilin zu spalten.

9. Verwendung eines Enzyms ausgewählt aus der Gruppe bestehend aus
(i) Oxidoreduktasen der E.C.-Klassen 1.4.-.- und 1.5.-.-, die CH-NH-Gruppen oder CH-NH2-Gruppen als Elektronendonor benutzen ,
(ii) Oxidoreduktasen der E.C.-Klasse 1.11.-.-, die Peroxide als Elektronendonor oder -akzeptor nutzen,
(iii) Oxidoreduktasen der E.C.-Klasse 1.14.-.-, die molekularen Sauerstoff einfügen oder reduzieren,
(iv) Oxidoreduktasen der E.C.-Klasse 1.18.-.- mit Eisen-Schwefel-Clustern, und
(v) Oxidoreduktasen der E.C.-Klasse 1.6.-.-, welche einen Cofaktor wie NADH oder NADPH verwenden;
Zur Spaltung sekundärer und/oder tertiärer Amine der allgemeinen Formeln (I) oder (II) wie in den Ansprüchen 3 und 4 definiert.
